# EUROPEAN PATENT SPECIFICATION

(11) **EP 2 226 395 B1**
(45) Date of publication and mention of the grant of the patent: **10.04.2013**
(21) Application number: 10154688.5
(22) Date of filing: 25.02.2010
(51) Int. Cl.: C12Q 1/68

(54) **Method and Kit for the detection of allergens**
Verfahren und Kit zur Erkennung von Allergenen
Procédé et Kit de détection d'allergènes

(30) Priority: 27.02.2009 IT RM20090092
(43) Date of publication of application: 08.09.2010
(73) Proprietor: Università degli Studi di Parma, 43100 Parma (IT)
(72) Inventor: Marmiroli, Nelson, 43100, Parma PR (IT); Gulli, Mariolina, 43100, Parma PR (IT); Pafundo, Simona, 43100, Parma PR (IT)
(74) Representative: Predazzi, Valentina

(56) References cited:
- ROSSI STEFANO ET AL: "A PNA-array platform for the detection of hidden allergens in foodstuffs" EUROPEAN FOOD RESEARCH AND TECHNOLOGY, vol. 223, no. 1, May 2006 (2006-05), pages 1-6, XP019328356 ISSN: 1438-2377
- ARLORIO ET AL: "Detection of hazelnut (Corylus spp.) in processed foods using real-time PCR" FOOD CONTROL, BUTTERWORTH, LONDON, GB, vol. 18, no. 2, 1 February 2007 (2007-02-01), pages 140-148, XP005572767 ISSN: 0956-7135
- BRZEZINSKI JENNIFER L: "Detection of cashew nut DNA in spiked baked goods using a real-time polymerase chain reaction method" JOURNAL OF AOAC INTERNATIONAL, AOAC INTERNATIONAL, ARLINGTON, VA, US, vol. 89, no. 4, 1 July 2006 (2006-07-01), pages 1035-1038, XP009123543 ISSN: 1060-3271
- BREZNA B ET AL: "A novel real-time polymerase chain reaction (PCR) method for the detection of walnuts in food" EUROPEAN FOOD RESEARCH AND TECHNOLOGY, vol. 223, no. 3, July 2006 (2006-07), pages 373-377, XP019420455 ISSN: 1438-2377
- DATABASE EMBL [Online] 9 March 2001 (2001-03-09), "Sesamum indicum 2S albumin mRNA, complete cds." XP002548823 retrieved from EBI accession no. EMBL:AF240005 Database accession no. AF240005
- DATABASE EMBL [Online] 14 June 2005 (2005-06-14), "Sequence 1 from patent US 6884877." XP002548824 retrieved from EBI accession no. EMBL:AR652779 Database accession no. AR652779 -& US 2003/124060 A1 (ROUX KENNETH H [US] ET AL) 3 July 2003 (2003-07-03)
- DATABASE EMBL [Online] 14 May 1996 (1996-05-14), "C.avellana gene for major allergen Cor a 1 (clone CAGC10)" XP002548826 retrieved from EBI accession no. EMBL:Z72439 Database accession no. Z72439
- DATABASE EMBL [Online] 24 June 2007 (2007-06-24), "TFR5_51_D11_E001.g1 USDA-Tifton Peanut Library TFR5 Arachis hypogaea cDNA clone TFR5-051-84_D11 5', mRNA sequence." XP002548825 retrieved from EBI accession no. EMBL:ES711138 Database accession no. ES711138
- NISHIWAKI MORIE ET AL: "Genotyping of human papillomaviruses by a novel one-step typing method with multiplex PCR and clinical applications." JOURNAL OF CLINICAL MICROBIOLOGY APR 2008, vol. 46, no. 4, April 2008 (2008-04), pages 1161-1168, XP002548819 ISSN: 1098-660X
- PIKNOVA L ET AL: "A novel real-time polymerase chain reaction (PCR) method for the detection of hazelnuts in food" EUROPEAN FOOD RESEARCH AND TECHNOLOGY, vol. 226, no. 5, March 2008 (2008-03), pages 1155-1158, XP002548820 ISSN: 1438-2377
- SCHOERINGHUMER KERSTIN ET AL: "Development and Validation of a Duplex Real-Time PCR Method To Simultaneously Detect Potentially Allergenic Sesame and Hazelnut in Food" JOURNAL OF AGRICULTURAL AND FOOD CHEMISTRY, vol. 57, no. 6, March 2009 (2009-03), pages 2126-2134, XP002548822 ISSN: 0021-8561
- HASHIMOTO HIROYUKI ET AL: "[Detection of allergenic substances in foods by a multiplex PCR method].", SHOKUHIN EISEIGAKU ZASSHI. JOURNAL OF THE FOOD HYGIENIC SOCIETY OF JAPAN OCT 2007 LNKD- PUBMED:18027545, vol. 48, no. 5, October 2007 (2007-10), pages 132-138, ISSN: 0015-6426

### SEQUENCE LISTING

<110> Università degli studi di Parma
<120> Kit for the detection of allergens
<130> BE998R
<160> 18
<170> PatentIn version 3.3
<210> 1
   <211> 20
   <212> DNA
   <213> Artificial
<220>
   <223> Forward primer for detection of Prunus dulcis
<400> 1
   gaatgtgaac gcccactcag 20
<210> 2
   <211> 21
   <212> DNA
   <213> Artificial
<220>
   <223> Reverse primer for detection of Prunus dulcis
<400> 2
   atctccgttc tcgttcacca c 21
<210> 3
   <211> 22
   <212> DNA
   <213> Artificial
<220>
   <223> Forward primer for detection of Corylus avellana
<400> 3
   caaaggtgac catgaggtgg at 22
<210> 4
   <211> 23
   <212> DNA
   <213> Artificial
<220>
   <223> Reverse primer for detection of Corylus avellana
<400> 4
   accttttctt tgccaccctt aat 23
<210> 5
   <211> 22
   <212> DNA
   <213> Artificial
<220>
   <223> Forward primer for detection of Juglans regia
<400> 5
   attcgcagaa gctggttcta ga 22
<210> 6
   <211> 22
   <212> DNA
   <213> Artificial
<220>
   <223> Reverse primer for detection of Juglans regia
<400> 6
   cttccagcga caaatgctag ag 22
<210> 7
   <211> 22
   <212> DNA
   <213> Artificial
<220>
   <223> Forward primer for detection of Anacardium occidentalis
<400> 7
   taatgggtcc gcctacaaag tt 22
<210> 8
   <211> 24
   <212> DNA
   <213> Artificial
<220>
   <223> Reverse primer for detection of Anacardium occidentalis
<400> 8
   gaccaaaaca ggaaacgaga aaaa 24
<210> 9
   <211> 22
   <212> DNA
   <213> Artificial
<220>
   <223> Forward primer for detection of Arachis hypogea
<400> 9
   gtgaccgtag caaatggcaa ta 22
<210> 10
   <211> 21
   <212> DNA
   <213> Artificial
<220>
   <223> Reverse primer for detection of Arachis hypogea
<400> 10
   gtaggaaatg aaaccggatg g 21
<210> 11
   <211> 21
   <212> DNA
   <213> Artificial
<220>
   <223> Forward primer for detection of Sesamum indicum
<400> 11
   tcaagaacaa ccatggcgaa g 21
<210> 12
   <211> 22
   <212> DNA
   <213> Artificial
<220>
   <223> Reverse primer for detection of Sesamum indicum
<400> 12
   tggtcacagt ggtggtgtag gt 22
<210> 13
   <211> 76
   <212> DNA
   <213> Prunus dulcis
<400> 13
   gaatgtgaac gcccactcag tggtgtacgt gatcagagga aatgctaggg ttcaggtggt 60
   gaacgagaac ggagat 76
<210> 14
   <211> 54
   <212> DNA
   <213> Corylus avellana
<400> 14
   caaaggtgac catgaggtgg atgcagagca tattaagggt ggcaaagaaa aggt 54
<210> 15
   <211> 81
   <212> DNA
   <213> Juglans regia
<400> 15
   attcgcagaa gctggttcta gatcaggaca tcatgcacca taataagggt ccctgttagc 60
   tctagcattt gtcgctggaa g 81
<210> 16
   <211> 57
   <212> DNA
   <213> Anacardium occidentalis
<400> 16
   taatgggtcc gcctacaaag ttttcttttt ctctttttct cgtttcctgt tttggtc 57
<210> 17
   <211> 87
   <212> DNA
   <213> Arachis hypogaea
<400> 17
   gtgaccgtag caaatggcaa taacagaaag agctttaatc ttgacgaggg ccatgcactc 60
   agaatcccat ccggtttcat ttcctac 87
<210> 18
   <211> 100
   <212> DNA
   <213> Sesamum indicum
<400> 18
   tcaagaacaa ccatggcgaa gaagctcgca ctcgcagcag tcctcctggt ggccatggta 60
   gcgttggcca gcgccacgac ctacaccacc actgtgacca 100

## Description

The present invention relates to a method and a kit allowing to detect the presence, in analysed samples, of material obtained from the allergenic species: almond *(Prunus dulcis),* hazelnut (*Corylus avellana),* cashew (*Anacardium occidentalis),* peanut (*Arachis hypogea*), walnut (*Juglans regia),* and sesame *(Sesamum indicum*).

### STATE OF THE PRIOR ART

According to known scientific data, food allergies exhibit a prevalence of 3-4% in adults and of 6% in pediatric age, appear more frequently in childhood and usually tend to disappear as years go by, yet can also manifest themselves for the first time in adulthood.

Generally, foods most frequently causing clinical manifestations of food allergy are: eggs, milk, fish, shellfish, peanuts, hazelnuts, soy, wheat, then followed by vegetables such as apple, walnut, almond, cashew, celery, tomato, banana, kiwi fruit, peach, carrot, pear.

In several countries indicating the components of a food is mandatory; however, even indicating the components may prove insufficient. In fact, in individual foods allergens may be found which are unforeseen, as not peculiar to the natural composition of the food and due to cross-contamination between foods, cross-reactivity between food allergens (e.g., between peanut and soy: in fact, both belong to the Leguminosae family), use of allergising substances indicated with a generic name or not declared on labels, incorrect indications of substances utilised for food preparation, nomenclatures that are complex and different in the various countries, transgenic foods, contaminations with intentional additives (preserving agents, colouring matters, thickening agents, etc.) or accidental additives (polluting substances, antibiotics for veterinary uses, pesticides, etc.).

For instance, soy is used in a great number of preparations as an additional ingredient, a stabilizer in cereal-based products and in meat. Gums of plant origin are used as thickening agents or stabilizers. Likewise, peanuts, wheat, eggs and milk are of common use as additional ingredients.

The treatment of food allergies and intolerances essentially consists in excluding the foods at issue from the diet.

This presupposes a knowledge of foods composition, needed to single out the substances at issue, above all when contained in an apparent form in foods, as additives, or when able to give cross reactivities, as is typical of different allergens.

For this reason, there is an ever-increasing demand for reliable and sensitive analytical methodologies for the analysis of food products, both for the reasons set forth above and in order to comply with labelling requirements for the presence of trace allergens according to European Standards (2000/13/EC, 2003/89/EC, 2006/142/EC). Molecular methodologies based on DNA analysis can ensure a high sensitivity, such as that required in cases in which there is no tolerability threshold for contamination, as in the case of allergens.

Various methodologies, such as conventional PCR, PCR-ELISA, and real-time PCR (Poms et al., 2004; Ehlert et al. 2008), are described in the state of the art for the identification of products obtained from allergenic species in foods.

The ideal analysis should allow to identify in a sample, even one of processed material such as foods, also cooked at high temperatures, plural allergenic ingredients with a single assay. Ideally, in fact, an assay enabling to identify at one time the presence or absence of plural allergenic components allows to save time, money, and also starting material on which the test is carried out.

Kits for the detection of allergenic species in foods, based on the use of fluorescent probes and allowing the detection of individual allergens, are commercially available from various companies (e.g., Incura, Celbio). Though individual assays for individual allergens are being marketed, currently available systems are limited in that they detect the presence or absence of one allergen per assay.

SYBR Green I is a fluorescent molecule which is able to intercalate with a high affinity to double-stranded DNA, in a sequence-nonspecific manner. Various works report the use of this molecule to identify a given DNA fragment according to its size and melting temperature (Tm) (Cubie et al. 2001).

There are some applications of this methodology in the medical field, to diagnose the presence of viruses (Ong et al. 2007), or to identify bacterial strains (Fan et al. 2007).

In the field of food analysis, SYBR Green use has been described to diagnose the presence of organisms or contaminating substances such as fungi (Brandfass and Karlovsky 2006), pathogens, GMOs (Hernández et al. 2003) and, only for some allergens without any indication of the sequences used, by Pafundo et al. 2008.

The advantage of using SYBR-Green lies above all in the fact that it is possible to carry out a multiplex analysis, with no need to carry out the analysis on a gel and with no need to use toxicant reagents (e.g., ethidium bromide). Various authors (Ririe et al. 1997) have described the option of distinguishing amplicons obtained by Real-Time PCR with SYBR Green on the basis of their Tm.

An application of multiplex real-time PCR with SYBR green has been described for GMOs detection (Hernández M, et al., 2003). In said article a duplex reaction was described in which two primer pairs were used in the same reaction mixture and led to the obtainment of two amplification products that were recognised on the basis of their different Tm.

In the work presented by Elizaquivel P, Aznar R, (2008) a triplex real-time PCR reaction with SYBR Green I is described in which it is obtained the concomitant amplification of three fragments that are distinguished on the basis of their different Tm to identify the presence of contaminant bacteria (Escherichia coli O157:H7, Salmonella spp. and Staphylococcus aureus) in foods.

Rossi et al. (Eur Food res Technol (2006) 223: 1-6) discloses a duplex PCR assay for the detection of both peanut and hazelnut in raw materials and commercial products. A PNA array device is used in combination with the duplex PCR for recognizing the PCR products.
Hashimoto et al.(Journal of the food hygienic society of Japan, vol. 48, no. 5, 2007, pages 132-138,2007) discloses a multiplex PCR method for the detection of DNAs of three allergenic substances (wheat, buckwheat and peanut) in food.

### SUMMARY OF THE INVENTION

The present description teaches a method and a kit allowing to detect, in a sample, the presence of material obtained from two or more of the species comprised in the group: almond *(Prunus dulcis),* hazelnut (*Corylus avellana*), cashew (*Anacardium occidentalis),* peanut (*Arachis hypogea),* walnut (*Juglans regia),* and sesame *(Sesamum indicum) .*

Methods are described herein which provide a multplex PCR reaction, allowing to concomitantly amplify two to six different species-specific DNA fragments (amplicons), when DNA from the target species is present in the sample subjected to amplification. Obtained amplicons are recognizable by analysis of the dissociation curve and/or of the related melting temperature (Tm), as each of them has a dissociation curve and a Tm recognizable from the other ones, or by analysis of the nucleotide sequence, as each amplicon has a size different from that of the other ones.

Therefore, the methods allow a very quick analysis in which up to six different allergenic species can be identified when present in a sample.

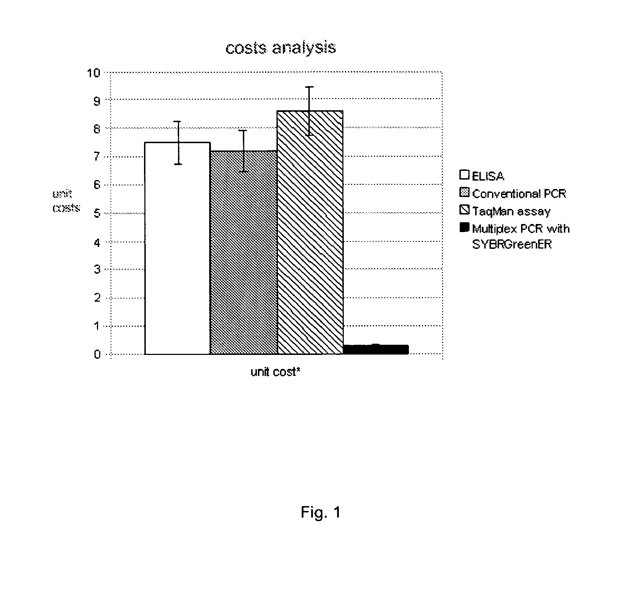

Taking into account that, as to the detection of allergens present in traces in foods, to date only protein or DNA detection-based analysis methods specific for a single allergen, or allergenic species, are available, it may be stated that with respect to the known state of the art a remarkable advancement has been achieved, associated, in specific embodiments, to a remarkable abatement of the costs per single reaction (see Fig. 1).

In the methods for the detection of the presence of an allergenic species, based on the detection of an allergenic protein, a certain number of false negatives can be had if the starting matrix on which the analysis is carried out has been subjected to a strong processing (thus being a complex matrix as defined herein) and therefore proteins are partly damaged. Therefore, the methods based on PCR use for the detection of allergenic species DNA allow greater sensitivity, as DNA is stabler during the processing. However, it should be borne in mind that in order to guarantee a high sensitivity the DNA fragments that are to be amplified must be of reduced dimensions (≤ 100bp) and the primers utilised must be highly specific, i.e. should amplify solely the target DNA segment. The option of carrying out screening analyses for the presence of allergens in samples to be analysed benefits from the use of multiplex systems.

However, a multiplex carrying out for such a complex analysis entails embodiment limitations rather evident to a person skilled in molecular genetics: by utilizing such a quick and sensitive detection system as PCR, the detection of different samples requires identification of oligonucleotides (primers) that do not pair thereamong, that may be used under the same reaction conditions, both as to the temperature and the length and number of cycles, as well as the ability not to give, under those given conditions, aspecific products. In particular, the method described in the present patent application allows a simultaneous detection of material obtained from six different allergenic species present, even in minimal amounts, in processed, cooked, packaged foods, more often than not introduced in said foods as products in turn processed (e.g., oils, flours, etc.) and stored at temperatures not particularly protecting DNA from degradation. The present description therefore discloses a selection of oligonucleotide sequences allowing amplification, even simultaneous, therefore with a single assay, of up to six different amplicons, each amplicon being species-specific and therefore indicative of the presence (when present) and absence (when absent) of material obtained from the species listed above.

In spite of all the unknown quantities, the inventors of the present invention were able to single out, for no less than six different species, sequences individually identifying each allergenic species (comprised in the group of almond, walnut, cashew, peanut, hazelnut and sesame), amplifiable by primers allowing their amplification, of highly species-specific amplicons under multiplex PCR conditions, even in foods having a complex matrix (i.e., comprising material processed and cooked even at high temperatures), each amplicon being further characterised in that it has a thermal dissociation curve of the double helix and a specific Tm different and recognizable from those of the other amplicons and a sequence length different from that of the other amplicons.

Therefore, objects of the disclosure are:
- a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed;
   b. amplifying said DNA with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   c. verifying the presence of species-specific amplicons and identifying them in the amplificate obtained in step b.;
- a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   b. verifying the presence of species-specific amplicons and identifying them in the amplificate obtained in step a.;
- a kit for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising one or more aliquots of primer pairs comprised in the group of SEQ ID 1 and 2, SEQ ID 3 and 4, SEQ ID 5 and 6, SEQ ID 7 and 8, SEQ ID 9 and 10, SEQ ID 11 and 12, and instructions for the identification of species-specific amplicons.

### AMPLICON

By "amplicon" it is meant, in the present description, a nucleotide fragment generated by means of PCR amplification of a nucleic acid.

### SENSE AND ANTISENSE PRIMER PAIR

Also defined as "sense and antisense oligonucleotide pair". By the terms "sense and antisense oligonucleotide or (primer) pair" it is meant a pair of oligonucleotides (primers for PCR) specifically designed to amplify a template sequence, therefore, given a template sequence, by "primer (or oligonucleotide) pair" for said sequence, a sense oligonucleotide and an antisense oligonucleotide are meant, designed to be used together in a PCR reaction to obtain the amplification of the desired template sequence.

### COMPLEX-MATRIX FOODS

Used herein to define a food product containing one or more given natural products or products obtained from certain food products (e.g., almond, hazelnut, cashew, peanut, walnut, and sesame) wherein said product/s has/have been subjected to processing (e.g., cooking, fractionation, etc.). Exemplary complex-matrix foods as intended herein may be biscuits, sweets, spirits, Nutella®, edible creams, pastry products, ice creams, etc..

### PRIMER COCKTAIL

By "primer cocktail" it is meant a mixture of different primer pairs used in the same PCR amplification.

### MELTING TEMPERATURE, Tm

Melting temperature (Tₘ) is defined as the temperature at which a population of double-stranded nucleic acid (e.g., dsDNA) molecules is 50% dissociated into single strands (*random-coil*). The melting temperature depends both on the total length of the DNA molecule and the specific sequence of the nucleotides.

### DISSOCIATION CURVE OR DISSOCIATION PROFILE

### (Also melting curve -Cm- or melting profile)

By "dissociation curve" or "dissociation profile" (or "melting curve" or "melting profile") it is meant the curve describing the degree of dissociation of duplex DNA/DNA or DNA/RNA strands in a given time period as a function of temperature.

Moreover, to the ends of the present description, by "dissociation curve" (and indicated aliases) it is also meant any representation of said curve (e.g., the dissociation derivative, etc.), commonly used by a technician in the art for a practical analysis of the dissociation curve or the Tm of a nucleic acid.

### MATERIAL OBTAINED FROM

By "material obtained from" one or a given species it is meant any material obtained from one or more parts of one or more individuals belonging to said species; in the present description reference is made to material obtained from plant (vegetable) species, therefore it is understood that the material may be obtained from any part of the plant (e.g., root apparatus, trunk, branches, leaves, flowers, seeds, fruits, etc.) by any process allowing to recover material from one or more parts of a plant (e.g., pressing, homogenizing, drying, powdering, crushing, cooking, and other processes commonly used to recover specific plant materials) or by any process for the production of specific vegetable products (e.g., oils, extracts, flours, meals, raw material, etc.). Therefore, by "obtained material" it is meant a material which, in spite of the treatments it has been subjected to, comprises anyhow the genome (and therefore the genetic material) of the species of interest. Hence, to the ends of the present description, by "material obtained from a certain species" it is meant any material obtainable from said species, as long as said material comprises the genome (and therefore the genomic DNA) of said species.

### CAPILLARY ELECTROPHORESIS

Various terms are used to indicate this technique: high performance capillary electrophoresis, HPCE, capillary zonal electrophoresis, CZE), free solution capillary electrophoresis, FSCE) and, finally, capillary electrophoresis, CE. Today, one of the most widespread terms is CE. By "capillary electrophoresis" to the ends of the present description it is meant the capillary electrophoresis specifically apt to separate nucleic acids on the basis of molecules' sizes with a small-size resolution.

### AMPLICON OBTAINED BY A PRIMER PAIR COMPRISED IN THE GROUP OF SEQ ID 1 and 2, SEQ ID 3 and 4, SEQ ID 5 and 6, SEQ ID 7 and 8, SEQ ID 9 and 10, SEQ ID 11 and 12

According to the present description, when mentioning an amplicon obtained by a primer pair comprised in the abovementioned group, or a mixture of said amplicons, it is meant an amplicon obtained by amplification of the target DNA for which said primer pairs are specific, or from genomic DNA, hence of the species for which an amplicon pair is specific, or from a fragment of such a DNA, cloned in a suitable vector (like plasmids indicated in the examples). Therefore, for the primer pair SEQ ID 1 and 2 the amplicon will have SEQ ID 13 or a natural allele of said sequence, for the primer pair 3 and 4 the amplicon will have SEQ ID 14 or a natural allele of said sequence, for the primer pair 5 and 6 the amplicon will have SEQ ID 15 or a natural allele of said sequence, for the primer pair 7 and 8 the amplicon will have SEQ ID 16 or a natural allele of said sequence, for the primer pair 9 and 10 the amplicon will have SEQ ID 17 or a natural allele of said sequence and for the primer pair 11 and 12 the amplicon will have SEQ ID 18 or a natural allele of said sequence.

### DETAILED DESCRIPTION OF THE FIGURES

**Fig. 1** Histogram representing a comparison of costs among analysis means for the detection of allergens in food samples with different techniques. The first three ones are known techniques i.e., ELISA □, conventional PCR ▧, TaqMan assay ■ and multiplex PCR with fluorescent SYBRGreenER intercalator ■.
   The figure demonstrates that the reaction according to the present description has, per each single allergen, a cost lower than 0.50 € for each reaction, therefore lower than 1 € for 2 amplicon pairs, lower than 1.5 € for 3 amplicons, etc.

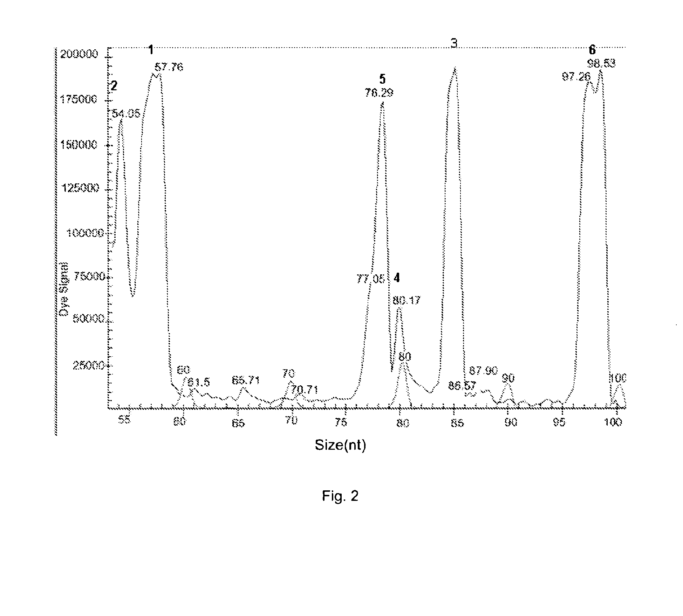

**Fig. 2** Analysis by capillary electrophoresis of the amplification product obtained from an equimolar mixture of six genomic DNAs in the presence of six primer pairs specific for each target. Each primer was labelled with Cy5, and the separation of the multiplex PCR product shows the presence of six DNA fragments of sizes corresponding to the predicted one, with a deviation of ± 2 bp. The height of the peaks is not correlated to the amount of each fragment. 1 Cashew, 2 Hazelnut, 3 Peanut, 4 Walnut, 5 Almond, 6 Sesame.

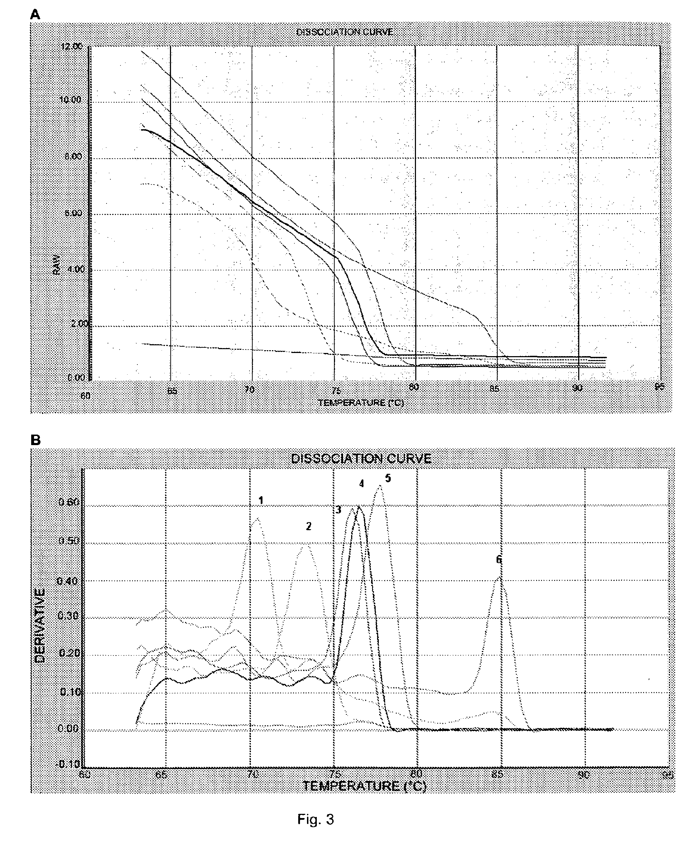

**Fig. 3** Results of multiplex Real-Time PCR reaction. The reaction was carried out with the instrument ABI Prism^{®} 7000 Sequence detection system. Six primer pairs were present in the reaction mixture and the analysed DNA was of an individual species. (1 Cashew, 2 Hazelnut, 3 Peanut, 4 Walnut, 5 Almond, 6 Sesame). Dissociation curves are shown overlapped. A) Dissociation curves (raw data); B) Dissociation curves obtained by calculation of the negative first-order derivative of the raw data (transformed data).

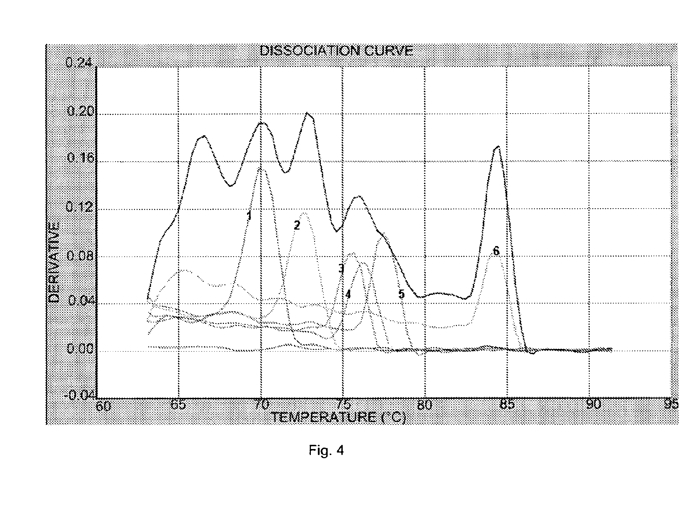

**Fig. 4** Multiplex Real-Time PCR analysis using SYBR^{®}GreenER™. The purple line (7) shows the negative first-order derivative of the dissociation curve of the amplification products, obtained by amplifying the mixture of six DNAs with the mixture of the six primer pairs. The coloured curves (1-6) show the derivatives of the dissociation curves obtained by amplifying the individual DNAs with the mixture of the six primer pairs. 1 Cashew, 2 Hazelnut, 3 Peanut, 4 Walnut, 5 Almond, 6 Sesame.

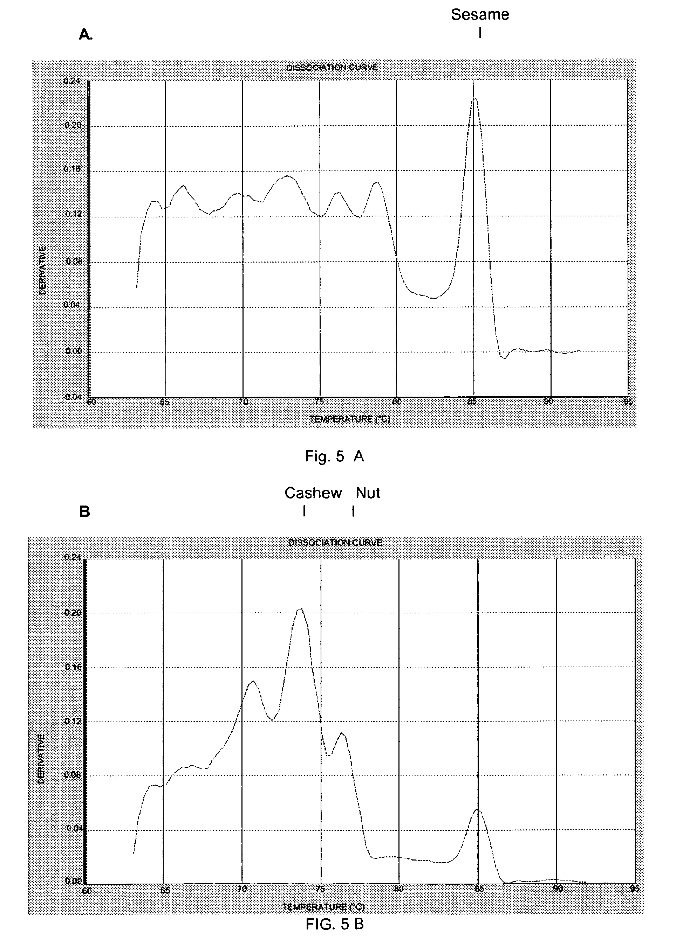

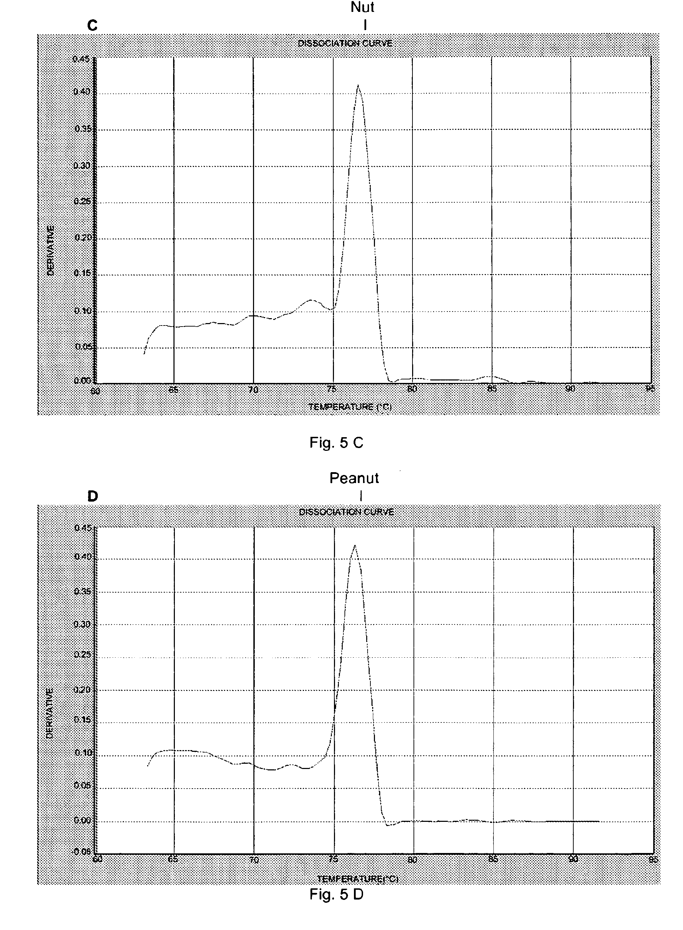

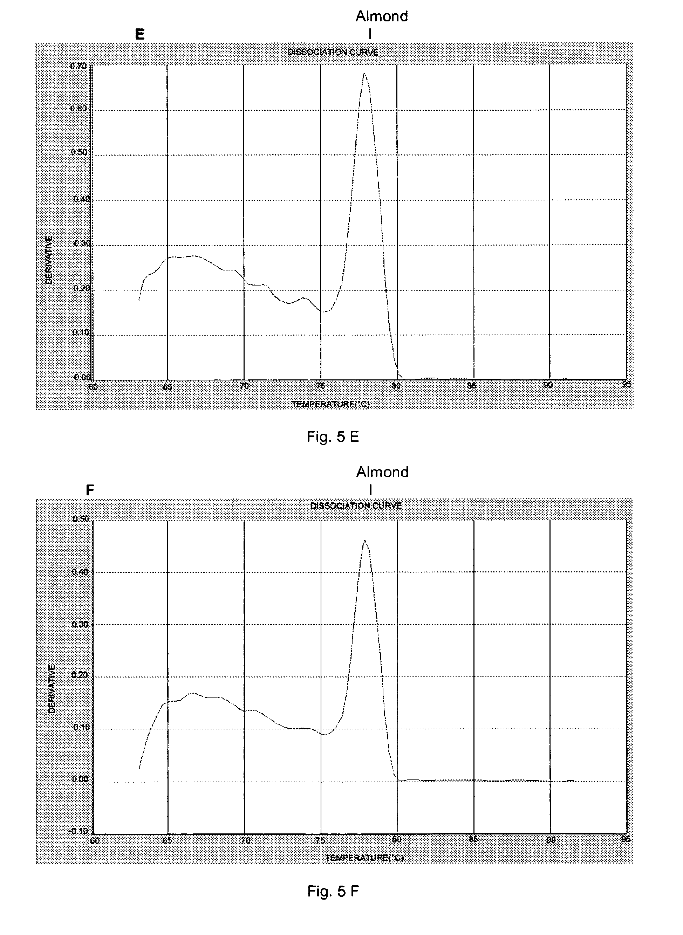

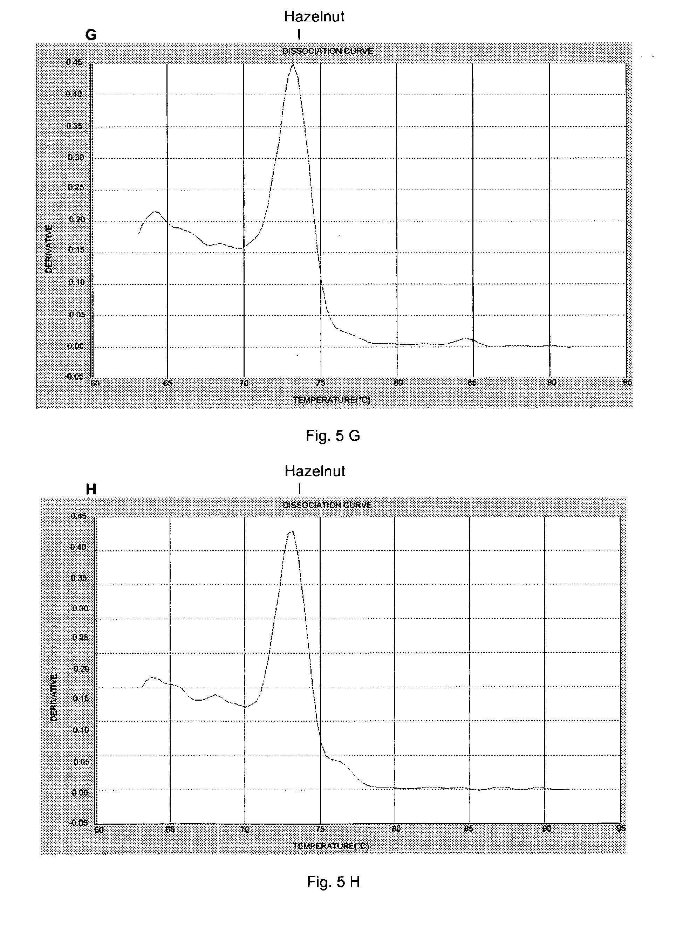

**Fig. 5****.** Multiplex Real-Time PCR using SYBR®GreenER™ on commercial products DNA. Each curve corresponds to the result of the amplification carried out on DNA extracted from single products. A) crackers with sesame and traces of nuts; B) biscuits with cashews and traces of nuts; C) biscuits with walnut and traces of nuts; D) chocolate with peanuts; E) amaretti biscuits; F) biscuits with almond flour and traces (of nuts); G) biscuits with hazelnuts and traces (of nuts); H) biscuits with hazelnut cream and traces (of nuts).

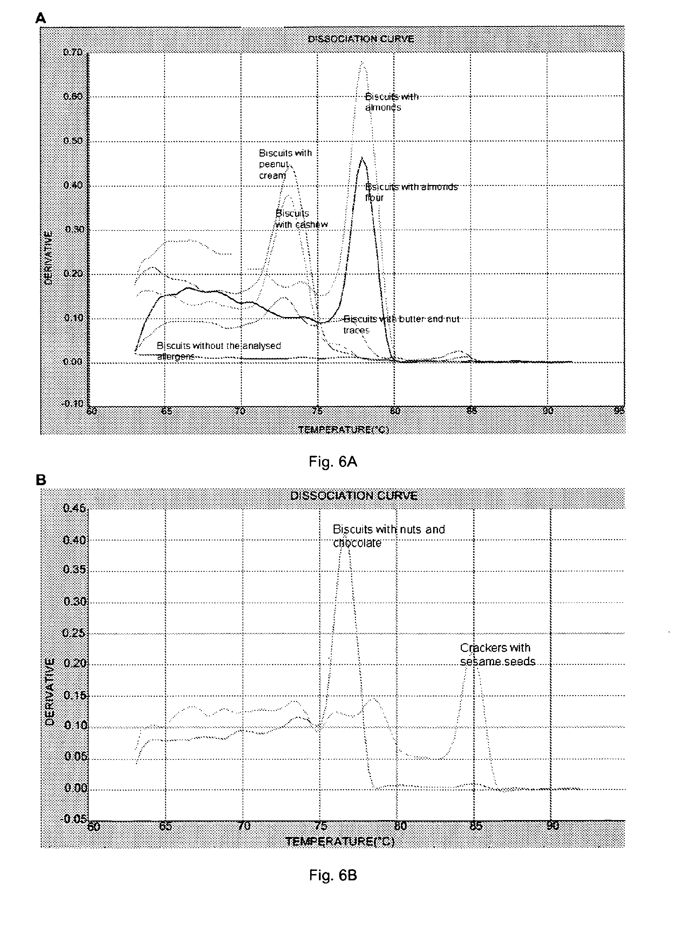

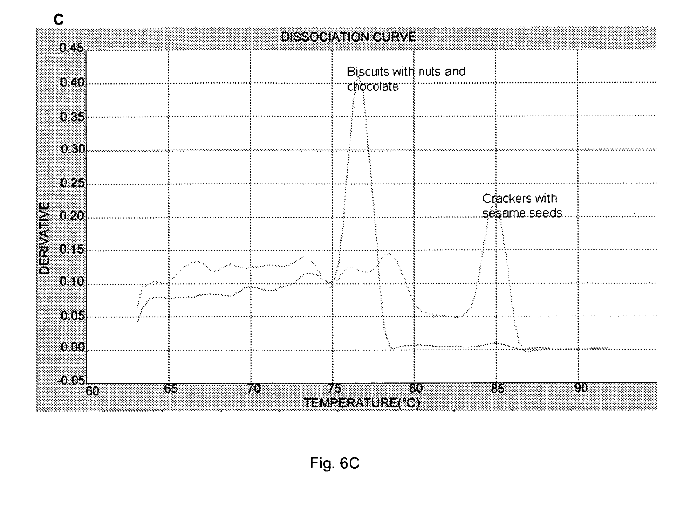

**Fig. 6****.** Multiplex Real-Time PCR using SYBR^{®}GreenER™ on DNA extracted from commercial products.

Each curve corresponds to the result of the amplification carried out on DNA extracted from individual products. Blue bars denote the Tₘ of the peak expected for each species.
A) DNA extracted from various biscuit types;
B) DNA extracted from crackers with sesame seeds and biscuits with walnuts and chocolate;
C) DNA extracted from chocolate with peanuts and biscuits with cashews and honey.

### DETAILED DESCRIPTION OF THE SEQUENCES

The Table below reports the sequences of the oligonucleotide pairs for species-specific amplification of material obtained from the species reported in the first column; in the second column the sequence of each primer and the number with which it is reported in the sequence list are indicated.

| **Species** | **Primer Sequences** |
|---|---|
| *Prunus dulcis* | SEQ ID NO. 1 |
| | FOR-GAATGTGAACGCCCACTCAG |
| | |
| | SEQ ID NO.2 |
| | REV-ATCTCCGTTCTCGTTCACCAC |
| *Coylus avellana* | SEQ ID NO. 3 |
| | FOR-CAAAGGTGACCATGAGGTGGAT |
| | |
| | SEQ ID NO. 4 |
| | REV-ACCTTTTCTTTGCCACCCTTAAT |
| *Juglans regia* | SEQ ID NO. 5 |
| | FOR-ATTCGCAGAAGCTGGTTCTAGA |
| | |
| | SEQ ID NO. 6 |
| | REV-CTTCCAGCGACAAATGCTAGAG |
| *Anacardium occidentalis* | SEQ ID NO. 7 |
| | FOR-TAATGGGTCCGCCTACAAAGTT |
| | |
| | SEQ ID NO. 8 |
| | REV-GACCAAAACAGGAAACGAGAAAAA |
| *Arachis hypogea* | SEQ ID NO. 9 |
| | FOR-GTGACCGTAGCAAATGGCAATA |
| | |
| | SEQ ID NO. 10 |
| | REV-GTAGGAAATGAAACCGGATGG |
| *Sesamum indicum* | SEO ID NO. 11 |
| | FOR-TCAAGAACAACCATGGCGAAG |
| | |
| | SEQ ID NO. 12 |
| | REV-TGGTCACAGTGGTGGTGTAGGT |

Hereinafter there are reported the sequences of the species-specific amplicons generated by amplification of genomic DNA belonging to the species indicated in the above Table, with the nucleotides reported in the above Table.
SEQ ID 13 *Prunus dulcis (*amplified with SEQ ID NO 1 and 2) 76 bp
SEQ ID 14 *Corylus avellana,* (amplified with SEQ ID NO 3 and 4) 54 bp
SEQ ID 15 *Juglans regia* (amplified with SEQ ID NO 5 and 6) 81bp
SEQ ID 16 *Anacardium occidentalis* (amplified with SEQ ID NO 7 and 8) 57bp
SEQ ID 17 *Arachis hypogea* (amplified with SEQ ID NO 9 and 10) 87bp
SEQ ID 18 *Sesamum indicum* (amplified with SEQ ID NO 11 and 12) 100bp

### DETAILED DESCRIPTION OF THE INVENTION

As indicated above, the present description relates to a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising steps of DNA amplification and analysis of obtained amplicons.

Oligonucleotides have been identified which are able to amplify in a highly species-specific manner, under the same PCR conditions and also in Multiplex-PCR (therefore useful in the form of nucleotide cocktails) given species-specific DNA fragments, when an amplification is carried out in the presence of template DNA belonging to the species for which they are specific. The DNA fragments obtained by amplification, i.e., amplicons, will then be synthesized by PCR only in the presence of material obtained from a precise species in the analysed sample.

As already defined above, the material obtained from a species will be material obtained from a species x (e.g., *Prunus dulcis, Corylus avellana, Juglans regia, Anacardium occidentalis, Arachis hypogea, Sesamum indicum*) said material comprising genomic DNA of said species x.

According to the present description the sample may be any one product, food, cosmetic, herbal or other, in which the products obtained from the above-indicated species may also be products derived from processes requiring high-temperature treatments (e.g., cooking). For instance, with regard to foods, the method and kit described herein allow the simultaneous detection of the presence of material obtained from two or more of the species comprised in the group: almond (*Prunus dulcis),* hazelnut (*Corylus avellana*), cashew (*Anacardium occidentalis),* peanut *(Arachis hypogea*), walnut (*Juglans regia*), and sesame *(Sesamum indicum),* also in complex food matrices.

The method of the present description may be carried out upon DNA extraction from the sample to be analysed, or directly on material collected from the sample diluted or suspended in the PCR mixture.

Of course, the yield of the method on extracted DNA is more effective, yet it is not always possible to efficiently extract DNA; in those cases, PCR could be carried out on the sample itself, suitably treated in order to be resuspended in the amplification mixture. In case the method is carried out directly on the sample without DNA extraction, it could be necessary to subject the sample to a denaturing pretreatment of a length greater than that of the treatment carried out for the amplification reaction of the extracted DNA, and optimize the reaction conditions (concentrations of primers, reagents and enzyme) so as to obtain an optimum yield.

The methods reported in the present description are all based on the use of selected primer pairs, on their high specificity and on the possibility of carrying out, with such primer pairs, multiple reactions; said multiple reactions produce species-specific amplicons having, moreover, features essential to the ends of the carrying out of the method. Such features make said amplicons recognizable among each other by different methodologies; therefore, their presence can be highlighted.

The differences are simply due to the possibility of carrying out a multiplex PCR, either directly on the material to be analysed or by extracting the DNA from said material, and to the putative methods of analysis of the obtained data.

As to the possibility of carrying out a Multiplex PCR either directly on the material to be analysed or by first extracting the DNA, in the second case the method will comprise a DNA extraction step absent in the other embodiment.

However, the principles of the method are clearly common to both embodiments.

Therefore, the method according to the present description could be generally described as:
- a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed
   b. amplifying said DNA with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   c. verifying the presence of species-specific amplicons and identifying them in the amplificate obtained in step b..

Or, in case it is opted not to carry out DNA extraction, as:
- a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   b. verifying the presence of species-specific amplicons and identifying them in the amplificate obtained in step a..

DNA could be extracted from samples to be analysed according to all methods known to a technician in the art and as described in several articles in the literature, online publications and on instruction sheets of kits suitable for the purpose.

In particular, for genomic DNA extraction from nut seeds and derived products the method described by Meyer, R, et al (1996) in the section "Materials and methods-Isolation and characterisation of nucleic acids" was applied.

As already specified, the amplicons produced by amplification of target DNA of the above-reported species-specific primer pairs have features which make their presence individually identifiable in the amplificate.

Therefore, in the presence of an amplificate of a sample containing all six allergenic species, the features of the amplicons obtained from the primer pairs described herein allow identification of each of them also in the mixture resulting from multiplex amplification.

Mainly, there are two rather quick and very sensible ways allowing the identification of species present in the amplificate (and therefore in the analysed sample) by identification of the above-indicated amplicons. In an embodiment, the composition of the amplificate, i.e. which amplicons are present therein, could be analysed by examination of the dissociation curve (in its preferred representation, e.g., negative first-order derivative) of the amplificate, comparing the obtained curve either to the curves of each amplicon or to curves obtained from known mixtures of said amplicons. In another embodiment the amplicons could be distinguished on the basis of their length (it also different for each amplicon), e.g., by capillary electrophoresis of the amplificate.

The amplicons of the present description, besides being species-specific, have each a thermal dissociation curve different from the other ones; there follows that the presence of such amplicons in the mixture resulting from the Multiplex PCR amplification reaction with two or more of the primer pairs (1 and 2, 3 and 4, 5 and 6, 7 and 8, 9 and 10, 11 and 12) described herein may be easily and distinctly detected by an analysis of the dissociation curve of such a mixture. Analysis of the dissociation curve obtained from the amplificate allows detection of the presence or absence of material obtained from the allergenic species searched in the sample.

The dissociation curve can be easily analysed by carrying out the above-mentioned PCR reaction in the presence of a fluorescent DNA intercalator, by subjecting the amplificates to gradual thermal denaturation and detecting the release of fluorescent intercalator (which occurs upon double-helix denaturation) measuring fluorescence intensity at the various temperatures. This step can be easily carried out by utilising a Real-Time PCR apparatus allowing this type of detection.

In short, in this embodiment the method may be described as:
- a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed (optional step)
   b. amplifying said DNA with two or more primer pairs comprised in the group of SEQ ID 1 and 2, SEQ ID 3 and 4, SEQ ID 5 and 6, SEQ ID 7 and 8, SEQ ID 9 and 10, SEQ ID 11 and 12, in the presence of a fluorescent DNA intercalator;
   c. verifying the presence of species-specific amplicons in the amplificate obtained in step b. by analysing the thermal dissociation curve of the amplificate and comparing it with the thermal dissociation curve of each isolated amplicon obtained by a primer pair comprised in the group indicated in step b., or with one or more thermal dissociation curves of known mixtures of amplicons obtained with two or more primer pairs indicated in step a., so as to detect whether one or more of said species is present in said samples.

DNA extraction at step a. could be carried out as discussed and described above.

In the embodiment without DNA extraction the method could synthetically be described as follows:
- a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   b. verifying the presence of species-specific amplicons and identifying them in the amplificate obtained in step a. by analysing the thermal dissociation curve of the amplificate obtained in step a. and by comparing it with the thermal dissociation curve of each isolated amplicon obtained by a primer pair comprised in the group indicated at step a., or with one or more thermal dissociation curves of known mixtures of amplicons obtained by two or more primer pairs indicated in step a.

Amplification could be carried out with two or more of the primer pairs described in any combination, (e.g., SEQ IDs : 1,2,3,4; 1,2,5,6; 1,2,7,8; 1, 2, 9, 10; 1, 2, 11, 12; 3, 4, 5, 6; 3,4,7,8; 3,4,9,10; 3,4,11,12; 5,6,7,8; 5,6,9,10; 5, 6, 11, 12; 7, 8, 9, 10; 7, 8, 11, 12; 9, 10, 11, 12; 1, 2, 3, 4, 5, 6; 1,2,3,4,7,8; 1,2,3,4,9,10; 1,2,3,4,11,12; 1,2,5,6,7,8; 1,2, 5, 6, 9, 10; 1, 2, 5, 6, 11, 12; 1, 2, 7, 8, 9, 10; 1,2,9,10,11,12; 1,2,3,4,5,6,7,8; 1, 2, 3, 4, 5, 6, 9, 10; etc.), therefore the method (and the kit) could utilise (or contain) all possible combinations with two, three, four, five and six primer pairs, selecting them in the group of SEQ ID 1 and 2, SEQ ID 3 and 4, SEQ ID 5 and 6, SEQ ID 7 and 8, SEQ ID 9 and 10, SEQ ID 11 and 12.

Multiplex PCR could be carried out in the presence of a fluorescent DNA intercalator that, concomitantly to double-helix dissociation of the amplicons, will be released, making the dissociation curve of the amplificate easily analysable. Such an intercalator could be any intercalator apt to be read by a machinery able to measure the dissociation curve of nucleic acids; among these, yet not limited thereto, are SYBR^{®}GreenER™, Fast SYBR^{®} Green, LCGreen Plus+, SYTO9, EVA Green, SYBR^{®} Green I.

To read the results, it will suffice to utilise the curves obtained during the stage of dissociation of the DNA mixture post-amplification, with respect to the curves obtained during the dissociation stage of the DNA of each single amplicon and/or of known mixtures of amplicons in all possible combinations with two, three, four, five or six amplicons (e.g., hazelnut and walnut, hazelnut and almond, hazelnut and sesame, hazelnut and cashew, hazelnut and peanut, almond and sesame, almond and cashew, almond and peanut, almond and walnut, sesame and walnut, sesame and cashew, sesame and peanut, walnut and cashew, walnut and peanut, almond, sesame and walnut, almond, sesame and hazelnut, almond, sesame and cashew, almond, sesame and peanut, almond, walnut and hazelnut, almond, walnut and cashew, almond, walnut and peanut, etc.).

Such curves can be easily prepared so as to make a graphical representation of all possible combinations, from two to six allergenic species, of the above-indicated allergenic species.

In order to easily make these curves, plasmids were prepared containing the target DNA of each primer pair; therefore, it is possible to prepare each mixture of amplicons simply by amplifying the desired plasmid DNA, and represent its dissociation curve in the preferred form (e.g., negative first-order derivative, or other forms making its reading and interpretation simple and effective). In this embodiment, it will be possible to utilise any representation of the dissociation curve of the amplificate, such as those generated by Real-Time PCR apparatuses, expressed as negative first-order derivative, and other representation forms of such a dissociation curve and of the Tm indicated by its point of inflection, commonly utilised in the field to compare different sequences thereamong.

In this way, evidently any known means for recognizing the amplicons of the invention, directly or indirectly correlated to the comparison of the respective dissociation curves or of the respective Tms, is an object of the present invention. Since many of said measurings are automatically carried out by laboratory machineries, in order to provide a sufficient description of the present invention it is not necessary to list all possible mathematical operations that may be performed on the dissociation curve or on the Tm value of a specific nucleotide sequence usually in a duplex form (es DNA/DNA, DNA/RNA, RNA/RNA). In fact, for the implementation of the present invention it will suffice to use apparatuses, such as Real-Time PCR or other ones, allowing a dissociation protocol, a fluorescence detection during said dissociation, the automatic computation and processing of the dissociation curves and/or the Tm of nucleotide sequences, and yielding data allowing to compare different sequences thereamong (see Fig. 3).

For instance, the dissociation curve can be obtained thanks to an instrument which is able to detect fluorescence emitted by a fluorophore. Fluorescence detection frequency during the dissociation stage is a specific feature of the instrument used. Therefore, after a conventional PCR occurred in the presence of a fluorophore intercalating the DNA double helix, the instrument should be able to start a "dissociation protocol", in which temperature is increased from 60°C to 95°C with an increase varying on different instruments; e.g., for a ABI Prism 7000 the increase is of 0.022°C/s, for an overall duration of 27 min. This in order to allow denaturation of the double helix of amplicons obtained during PCR and fluorophore release. Thanks to fluorescence detection, it will be possible to display the pattern of such kinetics as a curve that will undergo an inflection at the start of the denaturation, returning to linear when strands will be completely separated and the fluorophore released. Amplicon Tm is defined as the temperature at which 50% of the molecules have been denatured, and it corresponds to the median point of the curve inflection. Since reading the dissociation curves can prove difficult, they are usually transformed into their negative first-order derivative. Such a transformation allows to obtain curves whose peak corresponds to the amplicon Tm, and therefore to the median point of the dissociation curve inflection. In general, Real-Time PCR instruments can automatically perform such a transformation, though it is also possible to display the raw data (dissociation curve). In case PCR has yielded more amplicons as a result, this will be visible in the dissociation curves, with the presence of more inflections, and in the transformed curve, in the form of peaks at different temperatures.

The method described herein is based on the use of primers and reactions enabling a system sensitivity of up to about 5 pg for genomic DNA of almond, hazelnut and peanut, and of up to about 0.5 pg for genomic DNA of sesame, cashew and walnut, making the risk of detection of false negatives extremely remote.

In a further embodiment, the verification of the presence and the identification of the species-specific amplicons present in the mixture resulting from the amplification may be carried out by comparing the lengths of the amplicons present in said mixture.

A method of analysis of the above-mentioned mixture is represented in Fig. 2, wherein, following a Multiplex PCR with all primers from SEQ ID 1 to 12, labelled (in this case with Cy5, but any fluorochrome commonly used by a technician in the field is suitable), a capillary electrophoresis was carried out, allowing to clearly distinguish the sizes of the various amplificates and therefore clearly identify the presence of each species-specific amplicon in the mixture.

As can be seen in Fig. 2, amplicon 2 (Hazelnut, primer pair SEQ ID 3 and 4) has a length of about 54 nucleotides in the graph (known exact length: 54bp); amplicon 1 (Cashew, primer pair SEQ ID 7 and 8) has a length of about 58 nucleotides in the graph (known exact length: 57bp); amplicon 5 (Almond, primer pair SEQ ID NO 1 and 2) has a length of about 78 nucleotides in the graph (known exact length: 76bp); amplicon 4 (Walnut, primer pair SEQ ID NO 5 and 6) has a length of about 80 nucleotides in the graph (known exact length: 81bp); amplicon 3 (Peanut, primer pair SEQ ID NO 9 and 10) has a length of about 86 nucleotides in the graph (known exact length: 87bp); amplicon 6 (Sesame, primer pair SEQ ID NO 11 and 12) has a length of about 99 nucleotides in the graph (known exact length: 100bp).

Fig. 2 shows the extreme reliability of data analysis by capillary electrophoresis.

As mentioned above, amplicons length may be verified, e.g., by capillary electrophoresis techniques; these are highly sensitive techniques, therefore particularly suitable in those cases in which allergen traces have to be detected that are even lower than the protocol sensitivity indicated above for the analysis by dissociation curve. Moreover, such an analysis may be useful to confirm the presence/absence of a given amplicon in dubious cases, or when intending to verify with absolute accuracy the sequence of amplicons obtained by sample amplification.

According to the present description, capillary electrophoresis for establishing the length of amplicons present in the mixture resulting from Multiplex PCR may be carried out according to any implementation known to a technician in the field, apt to detect the length of a nucleotide sequence. For this reason, in the present description, at least one of the primers belonging to each pair used in the multiplex reaction is labelled with a fluorochrome suitable for DNA labelling (Cy2, Cy3, Cy5, various Cy dyes, rhodamine, fluorescein, and others known to a technician in the field) so as to be directly detected by apparatuses sensitive to fluorescence.

Capillary electrophoresis, in one of its embodiments, also allows DNA sequencing. Evidently, also the sequencing of an amplicon allows to determine its length (as well as its sequence) and allows a punctual identification of each amplicon present in the post-Multiplex PCR mixture according to the present description.

By "capillary electrophoresis" it is meant an electrophoretic technique envisaging the use of fused silica microcapillary tubing, with an internal diameter comprised between 10 and 100 microns, with a length between 30 and 50 cm. Said tubing is filled with a (gel-like) substance acting as a molecular sieve. The matrix may be polyacrylamide, dimethylacrylamide or other linear polymers, such as polyethylene oxide or hydroxyethyl cellulose.

To increase resolution, as is commonly known to a technician in the field, it is possible to act on: % of polymer used, run duration, voltage, temperature at which the run is performed. This type of electrophoresis can be used only in case a fluorescent labelling is available: at capillary level at a certain spot there will be a crack, through which a laser light will transit, able to excite the fluorochromes and induce a response that can be picked up by the detectors.

The samples to be analysed generally come from an amplification by PCR in the presence of a primer labelled with a fluorophore allowing to generate labelled amplicons. The instrument in which capillary electrophoresis occurs is equipped with a laser and a system allowing to detect fluorescence emitted by a fluorescent molecule. Two types of fluorescent molecules are present in the sample at issue: those of the amplicons and that due to a length marker exhibiting fragments of different and known length, labelled in a different way with respect to the amplicons. The detection system is able to detect and distinguish the two fluorescences. The length of the amplicon at issue is determined on the basis of the length of the marker fragments.

In short, the method in this case may be described as:
- a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed;
   b. amplifying said DNA with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12, wherein at least one primer of each pair is labelled with a fluorescent fluorochrome (dye);
   c. verifying the presence of species-specific amplicons and identifying them in the amplificate obtained in step b. by capillary electrophoresis and comparison of the sequence length of each isolated amplicon obtained by a primer pair comprised in the group indicated in step b.;
in case it is opted to carry out an extraction of the DNA of the sample as discussed above, or:
- a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12, wherein at least one primer of each pair is labelled with a fluorescent fluorochrome (dye);
   b. verifying the presence of species-specific amplicons and identifying them in the amplificate obtained in step a. by capillary electrophoresis and comparison of the sequence length of the sequences in the amplificate obtained in step a. and comparison with the sequence length of each isolated amplicon obtained by a primer pair comprised in the group indicated at step a.

In a further embodiment, the detection system could be represented by an electrophoresis of the amplificate on agarose gel or on polyacrylamide gel. The method, widely known in the literature, is always based on the principle of DNA charge and of an applied electrical field leading to DNA migration in a gel. As is known, migration rate is inversely proportional to the size of the migrating fragment; therefore, lighter fragments will migrate more rapidly than **heavier** ones. Usually, a reference molecular weight scale is used to establish the length of the DNA fragments that are to be analysed. In the specific case, the sequences of each amplicon being known, and means for cloning said sequences being available, it is possible to make an *ad hoc* scale, represented by all amplicons, enabling a direct comparison on gel.

In this case, the scale will have, both for the agarose gel and the polyacrylamide one, from the heavier band (nearer to the well) to the lighter one (farther from the well), the following bands of known molecular weight:
- 100bp (*Sesamum indicum*)
- 87bp (*Arachis hypogea)*
- 80bp (*Juglans regia*)
- 76bp (*Prunus dulcis*)
- 57bp (*Anacardium occidentalis)*
- 54bp (*Corylus avellana)*

For the carrying out of the method described herein with data analysis on agarose or acrylamide gel, it is evident that all techniques known to a person skilled in the art may be used, with no need of a more detailed description of how to prepare the gels and perform the run. In fact, to date such a technique is common knowledge to a person skilled in the art; concentrations of suitable agarose or polyacrylamide, buffer solutions for gel preparation, agarose or polyacrylamide concentrations, buffers for sample loading and gel staining systems are amply described in laboratory manuals, as well as in textbooks, and for a person skilled in the art require no inventive activity, nor undue experimenting.

For instance, it is known that for agarose gels a greater agarose concentration is matched by a better separation of low (<500bp) molecular weights and a poor separation of high (> 3kb) molecular weights; accordingly, in this embodiment agarose gel will be prepared at a concentration of at least 2%, and run times will be suitable for the separation of the molecular weights represented in the exemplary molecular weight scale above.

Moreover, for the carrying out of the method with data analysis and interpretation on agarose or polyacrylamide gel, any molecular weight marker enabling distinction of molecular weights of amplicons as reported in Table 2 could be used, without being fettered by the scale represented above.

Therefore, the method could synthetically be described as follows:
a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed;
   b. amplifying said DNA with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   c. verifying the presence of species-specific amplicons and identifying them in the amplificate obtained in step b. by electrophoresis on agarose or polyacrylamide gel.

Or as:
a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group SEQ ID 1 and 2, SEQ ID 3 and 4, SEQ ID 5 and 6, SEQ ID 7 and 8, SEQ ID 9 and 10, SEQ ID 11 and 12;
   b. verifying the presence of species-specific amplicons and identifying them.

In a further embodiment, presence verification and amplicon identification may be carried out by microarray.

The microarray slide ("chip") is a very powerful diagnostic instrument, able to single out, in one experiment, the presence/absence of a high number of targets. The chips exploit an important property of DNA, i.e. the pairing between complementary bases (T pairs with A and G with C) in its structure. The technique consists in the presence of a light signal (emitted by fluorophores at different wavelengths) at the hybridisation between the fluorophore-labelled target fragment and the corresponding probe attached (bonded) to the microarray slide. This bonding, with the entailed light emission, highlights that in the group of probes analysed there is the presence of a DNA fragment complementary to the probe that has "lit up" and, as a consequence, it allows to know the identity of the target fragment. Various (direct, indirect) techniques exist for labelling analysed DNA (or cDNA) through bonding with fluorophores emitting light at different wavelengths (generally in the red and green). The probe on the microarray slide is not bonded to fluorophores, therefore the microarray slide, non-hybridised and observed with a confocal scanner, shows no light signal.

In this embodiment of the method, the DNA of the sample to be analysed will be amplified, by PCR reaction as described above, utilising two or more specific primer pairs and the allergens indicated above, which will allow the amplification of fragments able to hybridise with the probes attached on the microarray slide.

The present description discloses a microarray able to simultaneously identify the presence or absence, in a sample, of each of at least 2 different allergens, up to each of the 6 different allergens described above from a sample. Therefore, such a microarray will be comprising, as probes attached on said microarray, nucleotide sequences comprised in the group of SEQ ID NO 1 to SEQ ID NO 18, or a fragment of the sequences 13 to 18, selected so as to detect the presence or absence of each of the above-indicated allergenic species.

The microarray according to the present description comprises, as probes spotted on the slide or on a suitable medium, one or more copies of probes having nucleotide sequences comprised in the group of SEQ ID NO 1 to SEQ ID NO 18, or fragments of the sequences from SEQ ID NO 13 to SEQ ID NO 18 on precise positions so as to form a microgrid, in order to simultaneously detect the presence or absence of material obtained from two or more species comprised in the group: *Prunus dulcis, Corylus avellana, Juglans regia, Anacardium occidentalis, Arachis hypogea, Sesamum indicum.* For each of said events, the sequences suitable for the detection and identification of each event are indicated in the detailed description of the sequences, sequences specific for each allergenic species mentioned above. As to the sequences comprised in the group of SEQ ID NO 13 to 18, said sequences in their entirety, or fragments thereof, could be used as identifying probes in the microarray of the present description. On average, the length of probes attached on a microarray may be comprised between 20 and 70 base pairs; accordingly, the lengths of SEQ ID NO 13-18 being comprised between 54 and 100 bases, it will be possible to use them in their entirety or it will be possible to use fragments thereof. A punctual description of the fragments, given the length of their sequences and their high species-specificity, is unnecessary. A technician in the field could easily select fragments of suitable consecutive nucleotides, ranging between 40 and 60 bases, or even shorter or longer ones, with no need to utilise inventive activity or undue experimenting. Evidently, the probes attached on the microarray could be complementary to any one of the two strands of sequences SEQ ID 13-18. An anchored control probe could be added to the microarray in order to verify the correct operation of the microarray technique.

Each probe could be anchored on the microarray in one or more copies (in the same site); clearly, the greater number of copies serves to increase the strength of the positive signal on the slide.

The microarray described herein will allow, according to the results obtained by hybridising the DNA obtained from amplification of (extracted or non-extracted) DNA comprised in the sample to be analysed, to identify the presence/absence of each of the 6 allergenic species indicated above.

The preparation of said microarray (i.e, synthesis of oligonucleotide probes and fastening of the latter on a suitable medium, e.g. a slide) is known to a technician in the field and requires no further indications in the present description. Microarray preparation may also be requested to companies providing this service. Therefore, further details on the preparation of the item *per se* are not necessary to the implementation of what is described in the present documentation.

In this embodiment, in the PCR reaction there could be used one or more primers per pair, labelled with a fluorochrome, or a labelled dNTP allowing hibridised amplicons visualization on the microarray. The probes having a specific position in the microarray, it is not necessary for the amplicons to be labelled with different fluorochromes; a control may be provided in one or more aliquots, respectively labelled with different fluorochromes, to give a possible orientation to the microarray. The microarray could otherwise comprise further orientation means (e.g., a knurling, a coloured zone, a mark, etc.) for result reading.

Probe hybridisation on the microarray with the DNA amplified by utilising two or more primer pairs described above, could be carried out according to protocols commonly utilised for such a step and known in the literature. For instance, obtained amplicons are suspended (e.g., after drying, or taking an aliquot of the PCR reaction), in a standard hybridisation buffer, (e.g.: 1.10 M NaCl, 0.11 M sodium citrate, 7 µg Salmon Sperm) subjected to denaturation and hybridised on the arrays described herein at a temperature approximately comprised between 55 °C and 65 °C, for a period of about 2 hours. At the end of the incubation, hybridisation solution is removed and arrays are rinsed with a suitable solution (e.g., 2X SSC (0.3 M NaCl, 0.03 M sodium citrate) and 0.1% SDS.

The microarray could then be read according to standard methodologies and compared, e.g., to a microgrid indicating the position of the probes representing each allergenic species on the microarray, so as to exactly identify the presence or absence of the allergenic species analysed in the amplified product.

The method in this embodiment could be resumed as follows:
a method for the simultaneous detection, in samples, of the presence of material obtained from two or more species comprised in the group: *Prunus dulcis, Corylus avellana, Juglans regia, Anacardium occidentalis, Arachis hypogea, Sesamum indicum,* comprising the following steps:
   a. extracting DNA from the sample to be analysed;
   b. amplifying said DNA with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   c. verifying the presence of species-specific amplicons and identifying them in the amplificate obtained in step b. by hybridisation on a microarray comprising, as probes spotted on said microarray, one or more copies of two or more oligonucleotides that are species-specific each for a different one of said species, having a sequence selected in the group comprising SEQ ID NO 1-18, and/or fragments of SEQ ID 13-18, of at least 20 nucleotides.

Or as:
a method for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising the following steps:
   a. amplifying the non-extracted DNA comprised in the samples with two or more species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12;
   b. verifying the presence of species-specific amplicons and identifying them by hybridisation on a microarray comprising, as probes spotted on said microarray, one or more copies of two or more oligonucleotides that are species specific each for a different one of said species, having a sequence selected in the group comprising SEQ ID NO 1-18, and/or fragments of SEQ ID 13-18, of at least 20 nucleotides.

In the present description there is also a kit for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising one or more aliquots of primer pairs comprised in the group of SEQ ID 1 and 2, SEQ ID 3 and 4, SEQ ID 5 and 6, SEQ ID 7 and 8, SEQ ID 9 and 10, SEQ ID 11 and 12; and means for the identification of species-specific amplicons.

As mentioned above, the kit could contain two, three, four, five or six among the above-indicated primer pairs, suitably subdivided into aliquots, and means for the identification of species-specific amplicons in the amplificate resulting from the use of said primer pairs. Such means could merely be direct and/or extrapolated (e.g., negative first-order derivative) representations of the individual dissociation curves of each amplicon obtainable by each above-indicated primer pair in the presence of the specific target DNA, and/or direct and/or extrapolated (e.g., negative first-order derivative) representations of dissociation curves of known mixtures of such amplicons, until providing direct and/or extrapolated (e.g., negative first-order derivative) representations of all possible combinations of the six amplicons.

Such data could also be provided in a computer-readable format, along with programs required to carry out any extrapolation from the dissociation curve and compare the provided data with those obtained following Multiplex PCR with the kit indicated.

Such means could also be aliquots of control DNA (e.g., genomic of each species or clones containing the fragment amplified from the primers for each species) in separate aliquots and/or in combinations that may be utilised as control and for the generation of dissociation curves made exactly with the same measuring means utilised on analysed samples.

Moreover, such means could also be graphical representations of capillary electrophoresis performed on individual amplicons and/or on known mixtures of such amplicons, as well as exact information on the size of each amplicon.

The kit could further contain Multiplex PCR reagents, one or more fluorescent DNA intercalators (as a non-limiting example, SYBR^{®}GreenER™, Fast SYBR^{®} Green, LCGreen Plus+, SYTO9, EVA Green, SYBR^{®} Green I).

In another embodiment, the kit could comprise aliquots of at least one primer of each of said primer pairs, labelled with a fluorescent fluorochrome (dye) (Cy2, Cy3, Cy5 and any other suitable fluorochrome commercially available and commonly known to a technician in the field).

The kit described herein could also comprise unlabelled aliquots of each primer and aliquots of at least one primer of each of said primer pairs, labelled with a fluorochrome, and means suitable for the detection and the singling out of amplicons present in the amplificate resulting from sample amplification with two or more of the primer pairs contained in the kit, both based on amplicons length and on dissociation curves as described above.

Therefore the kit could comprise one or more aliquots of reagents suitable for capillary electrophoresis, one or more aliquots of reagents suitable for agarose gel electrophoresis and/or one or more aliquots of reagents suitable for polyacrylamide gel electrophoresis, as well as one or more aliquots of molecular weight markers suitable for the detection of all amplicons represented by SEQ ID No 13-18, and/or indications on the length and sequence of each amplicon represented by SEQ ID No 13-18.

In an embodiment, the present description also discloses a kit for the simultaneous detection, in samples to be analysed, of the presence of material obtained from two or more species comprised in the group: *Prunus dulcis, Corylus avellana, Juglans regia, Anacardium occidentalis, Arachis hypogea, Sesamum indicum,* comprising one or more aliquots of primer pairs comprised in the group of SEQ ID 1 and 2, SEQ ID 3 and 4, SEQ ID 5 and 6, SEQ ID 7 and 8, SEQ ID 9 and 10, SEQ ID 11 and 12 and means for the identification of species-specific amplicons wherein such means is one or more microarrays as described above. The kit could contain 1 or more ready-to-use microarrays and the reading key of the microgrid representing the kit that allows to identify from each of at least 2 allergenic species to each of 6 allergenic species indicated above. The kit could further contain one or more aliquots for performing the hybridisation of the result of the amplification as described above, with the probes anchored on the slide.

Said microarray could comprise, as probes spotted thereon, one or more copies of two or more oligonucleotides species-specific for at least two of said species, selected in the group comprising SEQ ID NO 1-18, and/or fragments of at least 20 bases of SEQ ID 13-18.

Therefore, the microarray described herein will contain one or more copies of at least two oligonucleotides (each one specific for a different allergenic species among those described herein) selected in the group comprising SEQ ID NO 1-18, and/or fragments of at least 20 bases of SEQ ID 13-18. As explained in the foregoing, such fragments could be randomly selected in any position of SEQ ID 13-18 and could have a length of from about 20 bases to the entire sequence. The fragments will be made up of "subsequences" of SEQ ID NO 13-18 in which the nucleotides indicated in the starting sequences maintain their sequence one after the other; in fact, it is clearly preferable that said oligonucleotides exhibit no internal deletions with respect to the sequences from which they derive.

Moreover, the kit could further comprise suitable reagents for microarray hybridisation, such as, e.g., one or more aliquots of hybridization mix, one or more aliquots of sonicated SSDNA or the like, one or more aliquots of washing mix. Such mixes are known in the literature, and a non-limiting example thereof is also reported in the present description.

### EXAMPLES AND EXPERIMENTS

Examples aimed at better illustrating the methodologies disclosed in the present description are reported hereinafter; such examples are not to be considered in any way as a limitation to the preceding description and the subsequent claims.

Hereinafter, some examples are also reported of the selection techniques used in the carrying out of the methodology described herein in order to obtain primer pairs all concomitantly possessing the following characteristics:
- that
   a. were species-specific
   b. gave amplificates for each species, recognizable thereamong on the basis of their size
   c. gave amplificates for each species, recognizable on the basis of their dissociation curve
   d. were able to yield amplificates even on samples processed to the extreme, like, e.g., complex food matrices
   e. were amplificable by multiplex without mutually interacting, maintaining the same properties even when an amplicons mixture had to be analysed as a multiplex product.

All fragments (amplicons) obtained with these primers were sequenced, and the obtained sequence was verified to be identical to the expected one.

### 1. Determination of the theoretical Tm of each amplification product.

Starting from species-specific genes of each species, numerous primer pairs were designed in order to select the primer pairs that, at least theoretically, might meet the requisites needed for implementing the method and kit described herein.

To carry out a first selection of which might be primer pairs suitable for a recognition based also on the dissociation curve, primer pairs were selected, *a priori,* yielding, at least based on a computation of the theoretical Tm, products recognizable also on the basis of the Tm.

To simplify the reading, in the Table only primers claimed in the present application are reported, i.e., only those primer pairs that met not merely theoretical requirements, but also practical ones in order to implement the method described herein.

To this end, four different programs were used: Primer Express^{™} v.2.0 (Applied Biosystems), Biomath calculators (www.promega.com/biomath/calcll.htm), Oligo Calc (www.basic.northwestern.edu/biotools/oligocalc.html), Oligo Calculator (www.umdnj.edu/-humayun/oligo.html). The results of this analysis allowed to meet one of the main requirements for performing the planned analysis; in fact, as may be seen in Table 2, each amplicon has a different theoretical Tm value.

| **Table 2** | | | | | |
|---|---|---|---|---|---|
| **Species** | **Gene [Accession Number]** | **Primer** | **Primer Sequences** | **Amplicon length** | **Theoretical Tₘ *** |
| *Prunus dulcis* | Pru 1 (11s globulin) | PRU1 | SEQ ID NO1 FOR-GAATGTGAACGCCCACTCAG | 76 bp | 77°C |
| | [X78119] | | SEQ ID NO2 REV-ATCTCCGTTCTCGTTCACCAC | | |
| | | | | | |
| *Corylus avellana* | Cor a1 (Bet-v1 related protein) | CORA1 | SEQ ID NO3 FOR-CAAAGGTGACCATGAGGTGGAT | 54 bp | 73°C |
| | [Z72439] | | SEQ ID NO4 REV-ACCTTTTCTTTGCCACCCTTAAT | | |
| *Juglans regia* | Jug r1 (2s albumin) [U66866] | JUGR1 | SEQ ID NO5 FOR-ATTCGCAGAAGCTGGTTCTAGA | 81 bp | 76°C |
| | | | SEQ ID NO6 REV-CTTCCAGCGACAAATGCTAGAG | | |
| *Anacardium occidentalis* | Ana o1.0101 (vicilin-like protein) [AF395894] | ANAO1 | SEQ ID NO7 FOR-TAATGGGTCCGCCTACAAAGTT | 57 bp | 70°C |
| | | | SEQ ID NO8 REV-GACCAAAACAGGAAACGAGAAAAA | | |
| *Arachis hypogea* | Ara h1 (7s vicilin-like protein) [AF432231] | ARAH1 | SEQ ID NO9 FOR-GTGACCGTAGCAAATGGCAATA | 87 bp | 75.5°C |
| | | | SEQ ID NO10 REV-GTAGGAAATGAAACCGGATGG | | |
| *Sesamum indicum* | Ses i (2s albumin) [AF240005] | SESI | SEQ ID NO11 FOR-TCAAGAACAACCATGGCGAAG | 100 bp | 83°C |
| | | | SEQ ID NO 12 REV-TGGTCACAGTGGTGGTGTAGGT | | |

### 2. Verification of functionality of each primer pair

Each primer pair was utilised in a conventional PCR reaction in order to verify its specificity on the DNA of the reference plant species. There were selected only those primer pairs that exhibited high specificity for the species for which they had been designed. Amplification products sizes were verified by agarose gel analysis in the presence of ethidium bromide, or by capillary electrophoresis of the PCR product obtained by using primers labelled at 5' with fluorophore Cy5. Capillary electrophoresis was performed using the automatic sequencer CEQ™ 2000 XL Sequencer (Beckman-Coulter).

An example of the results obtained for the primer pairs selected herein is reported in Fig. 2.

Therefore, among them were selected the primer pairs that yielded amplificates recognizable thereamong on the basis of size.

### 3. Determination of the real Tm of each amplification product

There were selected only those primer pairs that exhibited high specificity for the species for which they had been designed and that yielded amplificates recognizable thereamong on the basis of their size.

To validate the Tm of each amplicon, a real-time PCR reaction was performed in the presence of SYBR^{®}GreenER™ (Invitrogen Corporation, Carlsbad, CA, USA). PCR reaction and analysis of dissociation curves were performed with the instrument ABI Prism^{®} 7000 Sequence detection system (Applied Biosystems division of Perkin-Elmer Corp., Foster City, CA). The reactions were performed in a final volume of 25 µl with 1x SYBR^{®}GreenER™qPCR SuperMix for ABI PRISM^{®} (Invitrogen Corp.)

There were selected only those primer pairs that yielded amplificates recognizable thereamong on the basis of the dissociation curve and related Tm.

The Tm obtained for primers from SEQ ID 1 to 12 are indicated in Table 3

**Table 3**

| SPECIES | *AMPLICON Tm* |
|---|---|
| Cashew (SEQ ID 7 and 8) | 70.5°C ±0.1 |
| Hazelnut (SEQ ID 3 and 4) | 73.3°C ±0.1 |
| Peanut (SEQ ID 9 and 10) | 75.9°C ±0.1 |
| Walnut (SEQ ID 5 and 6) | 76.3°C ±0.2 |
| Almond (SEQ ID 1 and 2) | 77.8°C ±0.2 |
| Sesame (SEQ ID 11 and 12) | 85.0°C ±0.2 |

### 4. Optimization of primer concentration for Multiplex PCR reaction

Primer concentrations initially used were of 250 nM for each primer pair (SEQ ID 1 to 12) and, based on results obtained, different combinations were tested: 100 nM, 150 nM, 200 nM, and 300 nM. PCR conditions were: 2 min at 50 °C, 15 min at 95°C; 40 cycles of 15 s at 95°C, 1 min at 60°C. Thereafter, a "Dissociation" step was carried out, during which the temperature was gradually increased from 60°C to 95°C (0.022°C/s). Obtained dissociation curves were converted into their negative first-order derivative; thus, a curve was obtained with a peak corresponding to the dissociation temperature (Tₘ) of the amplification product. This operation can be carried out automatically, by using the software present in the instrument, or fluorescence raw data may be exported and analysed with other data processing systems (e.g., MatLab)

Optimized concentrations were:

| | | |
|---|---|---|
| primer SESI | (SEQ ID 11 and 12) | 100 nM |
| primer ARAH1 | (SEQ ID 8 and 9) | 150 nM |
| primer CORA1 | (SEQ ID 3 and 4) | 150 nM |
| primer ANAO1 | (SEQ ID 7 and 8) | 150 nM |
| primers PRU1 | (SEQ ID 1 and 2) | 250 nM |
| primer JUGR1 | (SEQ ID 5 and 6) | 250 nM |

### 5. Multiplex analysis on DNA samples of single species and on DNA samples comprised of mixtures.

The optimized primer mixture was used to analyse Genomic DNA of each of the species subject of study. In that case, the result of the analysis consisted in obtaining a dissociation profile with a single peak corresponding to the Tm expected for the amplicon of the DNA of the species used, therefore:

**Table 4**

| *SPECIES* | *AMPLICON Tm* |
|---|---|
| Cashew | 70.5°C±0.1 |
| Hazelnut | 73.3°C±0.1 |
| Peanut | 75.9°C±0.1 |
| Walnut | 76.3°C±0.2 |
| Almond | 77.8°C±0.2 |
| Sesame | 85°C±0.2 |

The mixture of the six primer pairs having optimized concentrations was utilised to analyse genomic DNA mixtures prepared by mixing in a laboratory the DNA of each of the species subject of study in equal proportions (16.66% of each DNA). In that case the result of the analysis consisted in obtaining a dissociation profile with 6 peaks each at the Tm expected for the amplicon of each species present in the mixture (Fig. 3).

### 6. Multiplex analysis utilising different instruments and different intercalator molecules

A real-time Multiplex PCR reaction in the presence of SYBR^{®}GreenER™ was performed by utilising two different instruments:
1 - ABI Prism^{®} 7000 Sequence detection system
2 - Applied Biosystems 7900 HT Fast Real-Time PCR System

Both instruments are of the Applied Biosystems division Company of Perkin-Elmer Corp., Foster City, CA.

The reactions were performed in a final volume of 25 µl, in the presence of different types of intercalator molecules:
➢ SYBR^{®}GreenER™qPCR SuperMix.
➢ LCGreen^{®} Melting Dye (Idaho Technology inc., Utah, USA),
➢ Fast SYBR^{®} Green Master Mix (Applied Biosystems),
Fast PCR conditions were: 20 s at 95 °C, 40 1-s cycles at 95°C, 20 s at 60°C.

The PCR program was followed by a Dissociation protocol, in which temperature increased gradually from 60°C to 95°C (0.014°C/s for the Applied 7900HT instrument).

Dissociation curves were automatically converted into negative first-order derivative by the software present in the instrument.

With the ABI 7000 instrument the dissociation ratio was of 0.022°C/s. By using ABI 7000, discrimination of all amplicons was achieved (Fig. 4).

### 7. Determination of method sensitivity and LOD

The mixture of the six primer pairs with optimized concentrations was utilised to analyse six different mixtures of genomic DNA contained in a total of 50 ng DNA, prepared by mixing in laboratory the DNA of each of the species subject of study in the following proportions: 1% of one species and 99% of the other five species in equal proportions (19.8% each).

Moreover, each mixture was diluted 1:10, 1:100 and 1:1000, so as to contain an amount of total DNA of 5 ng, 0.5 ng and 0.05 ng, whereas the amount of DNA of the species present at 1% was of 50 pg, 5 pg and 0.5 pg. In that case the result of the analysis consisted in obtaining a dissociation profile with 6 peaks, each one at the Tm expected for the amplicon of each species present in the mixture (Table 5).

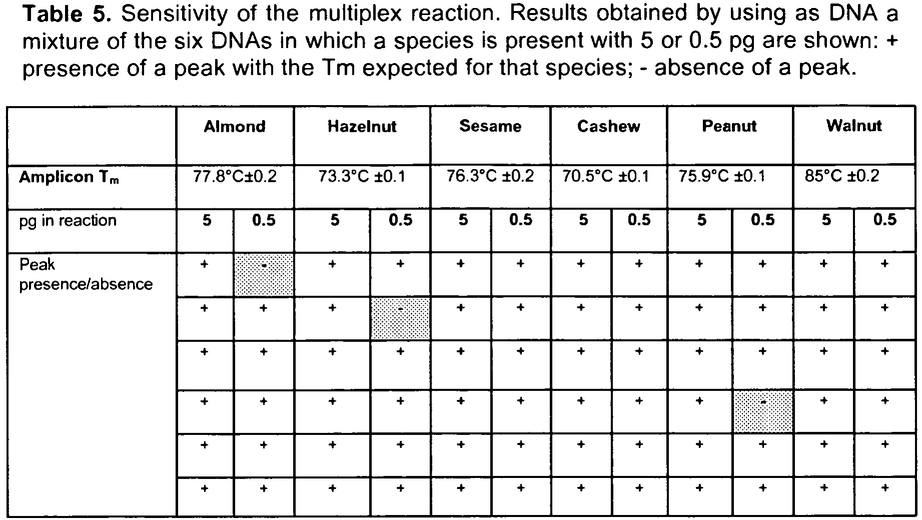

From the analysis performed it can be stated that the sensitivity of the optimised system is of about 5 pg for almond, hazelnut and peanut species, and of about 0.5pg for sesame, cashew and walnut species.

### 8. Production of recombinant plasmids containing each PCR product

The amplification products obtained with each primer pair (Table 1) were purified and cloned in the vector pGEM^{®}-T Easy (Promega, Madison, WI, USA). Plasmid DNA extracted from transformed recombinant cells was isolated, purified and sequenced according to conventional methodologies.

Three independent sequencings were performed for each clone, and the results analysed with the BLASTN program (http://www.ncbi.nlm.nih.gov/BLAST). These analyses confirmed the correspondence between cloned sequences and the sequences present in the database.

Plasmids were then used to obtain calibration curves: the plasmids were diluted so as to obtain 10⁶, 10⁵ and 10⁴ plasmid copies. Various mixtures of plasmids were prepared as indicated, each in alike amount, and such a mixture was serially diluted to have 10⁶, 10⁵ and 10⁴ copies of each plasmid.

These mixtures of plasmid DNA were used to perform Real-Time Multiplex PCR analyses with SYBR^{®}GreenER™, as described.

Each reaction yielded dissociation curves as expected; moreover, Ct value for each sample was evaluated. Obtained results served to obtain standard curves, relating the log₁₀ of the concentration of DNA with the Ct value. Such curves demonstrate reaction efficiency to be at least 99%.

Plasmids thus developed can be used to make a useful standard, which can be utilised also for purposes of absolute quantitation of the amount of target present in the reaction.

### 9. Development of a conventional array

For the development of this type of array platform, a fluorescent primer labelled with fluorophore Cy5 was utilised. The probe was designed so as to be highly specific for the amplicons of the invention, (e.g., by utilising the software OLIGOARRAY v.2.1 Nucleic Acids Research, 2003; 31, 12: 3057-3062). Parameters were set as reported hereinafter:
Melting temperature: 75 - 95°C
%GC: 40 - 60%
Maximum melting temperature (to avoid secondary structure-forming): 45°C

### Probe deposition and on-glass spotting

Depositions were carried out by utilising the instrument GMS 417 Arrayer (Genetic MicroSystems), and, e.g., SCHOTT Nexterion Slide A - type slides were used.

### Deposition conditions.

The oligonucleotides (probes) to be deposited can be diluted in 100 mM carbonate buffer at pH 9 and 0.01% SDS, to a final concentration of about 20 µM; subsequently 20 µL of each probe thus prepared was inserted into the well assigned thereto in a microtiter plate. The instrument was set so that each needle performs two touchings on the glass per each spot, spacing them of 300 µm one another and carrying out three repetitions. Deposition occurs at a temperature of 25°C and under 55% humidity conditions.

### On-glass oligonucleotide spotting conditions.

Glasses surface was rehydrated with steam for about 2-3 s. Subsequently, the glasses were placed for 10 s on a plate heated to a temperature of 80°C and exposed to UV radiation (800 mJ).

### Array hybridization with PCR products

### Pre-hybridization conditions.

Slides were dipped into the pre-hybridization solution for abt. 2 h at abt. 35°C; then, about 4 washings were performed, in deionized water at abt. 21°C for some seconds. The slides may be dried by a 3-min centrifugation at 200 rcf. The pre-hybridization solution was comprised of: 5X SSC, 0.1% v/v SDS, 1.5% w/v BSA (Sigma), 0.05 mg/ml herring sperm DNA, sterile bidistilled water to bring to a final volume of 100 ml.

### Hybridization conditions.

Hybridization conditions were optimized by using 15 µl of 50 µl of the PCR product, mixed with 5X SSC, 0.005% herring sperm DNA, to reach a final volume of hybridization solution equal to 70 µl. The solution was denaturated at 94°C for 4 min, and placed on ice right away to arrest denaturation of the double helix of the DNA fragments. The hybridization solution was then inserted into Camlab hybridization chambers, positioned beforehand on the deposition zones of the slides (22x25 mm ERIE slide covers). The slides thus prepared were then placed for 2 h 30' in a thermal incubator at 50-65°C.

### Washings.

Hybridization chambers were removed, and slides subjected to 4 washings at 21°C:
Washing 1: the slides were placed in a solution containing 2X SSC and 0.1% SDS at room temperature for 5 min.
Washing 2: the slides were transferred into a solution containing 2X SSC at the temperature of 42°C for 5 min.
Washing 3: the slides were inserted into a solution containing 0.2X SSC at room temperature for 1 min.
Washing 4: the slides were rinsed with a solution containing 0.05X SSC at room temperature for some seconds.

Finally, the slides were dried by a 3-min centrifugation at 200 rcf.

### Image acquisition

Slides images are acquired by scanner (GMS 418 Array Scanner) at various laser power and detector gain levels for each channel, so as to single out even the weakest signal.

### REFERENCE

Brandfass C. and Karlovsky P. 2006. Simultaneous detection of Fusarium culmorum and F. graminearum in plant material by duplex PCR with melting curve analysis. BMC Microbiology 6:4 doi: 10.1186/1471-2180-6-4
Bre 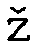 ná B., Hudecová L., Kuchta T. 2006. A novel Real-time polymerase chain reaction (PCR) method for the detection of walnuts in food. Eur. Foo Res. Technol. 223: 373-377.
Cubie H.A., Seagar A.L., McGoogan E., Whitehead J., Brass A., Arends M.J., Whitley M.W. 2001. Rapid real time PCR to distinguish between high risk papillomavirus types 16 and 18. J. Clin. Pathol.: Mol. Pathol. 54: 24-29.
Elizaquível P, Aznar R, 2008 A multiplex RTi-PCR reaction for simultaneous detection of Escherichia coli O157:H7, Salmonella spp. and Staphylococcus aureus on fresh, minimally processed vegetables. Food Microbiol. 25:705-713
Ehlert A., Hupfer C., Demmel A., Engel K.-H., Busch U. 2008. Detection of cashew nut in foods by a specific Real-time PCR method. Food Anal Methods 1: 136-143.
Fan W., Hamilton T., Webster-Sesay S., Nikolich M.P., Lindler L.E. 2007. Multiplex real-time SYBR Green I PCR assay for detection of tetracycline efflux genes of Gram-negative bacteria. Mol. Cell. Probes 22: 245-256.
Hernández M., Rodríguez-Lázaro D., Esteve T., Prat S., Pla M. 2003. Development of melting temperature-based SYBR Green I polymerase chain reaction methods for multiplex genetically modified organism detection. Anal. Biochem. 323: 164-170.
Meyer, R, Chardonnens F, Hübner P, Lüthy J. 1996. Polymerase chain reaction (PCR) in the quality and safety assurance of food: detection of soya in processed meat products. Z. Leben. Unters Forsch A, 203, 339-344.
Ong W.T., Omar A.R., Ideris A., Hassan S.S. 2007. Development of a multiplex real-time PCR assay using SYBR Green I chemistry for simultaneous detection and subtyping of H9N2 influenza virus type A. J.Virol. Met. 144: 57-64.
Pafundo S., Gulli M., Marmiroli N. SYBR®GreenER™ Real-Time PCR to detect almond in traces in processed food, submitted 2008.
Poms, R.E., Klein C.L., Anklam, E. 2004. Methods for allergen analysis in food: a review. Food Add. Contam. 21: 1-31.
Ririe K.M., Rasmussen R.P., Wittwer C.T. 1997. Product differentiation by analysis of DNA melting curves during the polymerase chain reaction. Anal. Biochem. 245: 154-160.

## Claims

1. A method for the simultaneous detection, in samples, of the presence of material obtained from *Prunus dulcis, Corylus avellana, Juglans regia, Anacardium occidentalis, Arachis hypogea* and *Sesamum indicum,* comprising the following steps:
a. extracting DNA from the sample to be analysed;
b. amplifying said DNA with six species-specific primer pairs comprised in the group of SEQ ID 1 and 2, SEQ ID 3 and 4, SEQ ID 5 and 6, SEQ ID 7 and 8, SEQ ID 9 and 10, SEQ ID 11 and 12 said amplification being carried out in the presence of a fluorescent DNA intercalator;
c. verifying the presence of species specific amplicons and identifying them in the amplificate obtained in step b by analysing the thermal dissociation curve of the amplificate obtained in step b. and by comparing the thermal dissociation curve of each isolated amplicon obtained by a primer pair comprised in said group or with one or more thermal dissociation curves of known mixtures of amplicons obtained by a primer pair comprised in said group.

2. A kit for the simultaneous detection, in samples to be analysed, of the presence of material obtained from *Prunus dulcis, Corylus avellana, Juglans regia, Anacardium occidentalis, Arachis hypogea* and *Sesamum indicum,* comprising one or more aliquot of six primer pairs of SEQ ID 1 and 2, SEQ ID 3 and 4, SEQ ID 5 and 6, SEQ ID 7 and 8, SEQ ID 9 and 10, SEQ ID 11 and 12; and means for the identification of species specific amplicons comprising one or more representation of the thermal dissociation curve of each isolated amplicon obtained by a primer pair comprised in said group, and/or one or more of representation of one or more thermal dissociation curves of known mixtures of amplicons obtained by six primer pair comprised in said group.

3. A kit, according to claim 2 further comprising a fluorescent DNA intercalator.

4. A kit, according to claim 3 wherein said a fluorescent DNA intercalator is selected between SYBR^{®}GreenER™, Fast SYBR^{®} Green, LCGreen Plus+, SYTO9, EVA Green, SYBR^{®} Green I.

5. A kit according to any one of claims from 2 to 4, further comprising one or more aliquot of PCR reagents.

## Patentansprüche

1. Verfahren zur simultanen Bestimmung der Anwesenheit von Material, das von *Prunus dulcis, Corylus avellana, Juglans regia, Anacardium occidentalis, Arachis hypogea und Sesamum indicum* erhalten wurde, in Proben, umfassend die folgenden Schritte:
a. Extraktion von DNA aus der zu untersuchenden Probe,
b. Amplifikation der DNA mit sechs speziesspezifischen Primerpaaren aus der Gruppe von SEQ ID 1 und 2, SEQ ID 3 und 4, SEQ ID 5 und 6, SEQ ID 7 und 8, SEQ ID 9 und 10 und SEQ ID 11 und 12, wobei die Amplifikation in der Anwesenheit eines fluoreszierenden DNA-Interkalators durchgeführt wird,
c. Bestätigung der Anwesenheit von speziesspezifischen Amplikons und Identifikation dieser im durch Schritt b erhaltenen Amplifikat mittels einer Analyse der thermalen Dissoziationskurve des durch Schritt b erhaltenen Amplifikats und durch den Vergleich der thermalen Dissoziationskurve jedes isolierten Amplikons, das mit Hilfe eines der Primerpaare aus der Gruppe erhalten wurde, oder mit einer oder mehreren thermalen Dissoziationskurve(n) von bekannten Amplikon-Gemischen, die unter Zuhilfenahme eines Primerpaars aus der Gruppe gewonnen wurden.

2. Kit zur simultanen Bestimmung der Anwesenheit von Material, das von *Prunus dulcis, Corylus avellana, Juglans regia, Anacardium occidentalis, Arachis hypogea und Sesamum indicum* erhalten wurde, in zu untersuchenden Proben, umfassend ein oder mehrere Aliquot(e) von sechs Primerpaaren der SEQ ID 1 und 2, SEQ ID 3 und 4, SEQ ID 5 und 6, SEQ ID 7 und 8, SEQ ID 9 und 10, SEQ ID 11 und 12, sowie Mittel zur Identifikation von speziesspezifischen Amplikons umfassend einen oder mehrere Repräsentanten der thermalen Dissoziationskurve jedes isolierten Amplikons, das unter Zuhilfenahme eines Primerpaars aus der Gruppe erhalten wurde, und/oder einen oder mehrere Repräsentanten einer oder mehrerer thermalen/thermaler Dissoziationskurve(n) von bekannten Amplikon-Gemischen, die unter Zuhilfenahme von sechs Primerpaaren aus der Gruppe gewonnen wurden.

3. Kit nach Anspruch 2, das außerdem einen fluoreszierenden DNA-Interkalator umfasst.

4. Kit nach Anspruch 3, wobei der fluoreszierende DNA-Interkalator ausgewählt ist aus SYBR®GreenER™, Fast SYBR® Green, LCGreen Plus+, SYTO9, EVA Green und SYBR® Green I.

5. Kit nach einem der Ansprüche 2 bis 4, das außerdem ein oder mehrere Aliquot(e) an PCR-Reagenzien enthält.

## Revendications

1. Procédé permettant de détecter simultanément la présence, dans des échantillons, de substances issues de *Prunus dulcis, Corylus avellana, Juglans regia, Anacardium occidentalis, Arachis hypogea* et *Sesamum indicum,* lequel procédé comporte les étapes suivantes :
a) extraire de l'ADN de l'échantillon à analyser ;
b) amplifier cet ADN à l'aide de six paires d'amorces spécifiques d'une espèce, constituant l'ensemble des paires d'amorces de Séquences N° 1 et N° 2, de Séquences N° 3 et N° 4, de Séquences N° 5 et N° 6, de Séquences N° 7 et N° 8, de Séquences N° 9 et N° 10, et de Séquences N° 11 et N° 12, cette amplification étant réalisée en présence d'un composé fluorescent s'intercalant dans l'ADN ;
c) et vérifier la présence d'amplicons spécifiques d'une espèce, et les identifier dans le produit d'amplification obtenu dans l'étape (b) en analysant la courbe de dissociation thermique de ce produit d'amplification obtenu dans l'étape (b) et en la comparant avec la courbe de dissociation thermique de chaque amplicon isolé obtenu à l'aide d'une paire d'amorces faisant partie dudit ensemble ou avec une ou plusieurs courbe(s) de dissociation thermique de mélanges connus d'amplicons obtenus à l'aide d'une paire d'amorces faisant partie dudit ensemble.

2. Trousse conçue pour la détection simultanée de la présence, dans des échantillons à analyser, de substances issues de *Prunus dulcis, Corylus avellana, Juglans regia, Anacardium occidentalis, Arachis hypogea* et *Sesamum indicum,* laquelle trousse comporte une ou plusieurs fraction(s) aliquote(s) des six paires d'amorces de Séquences N° 1 et N° 2, de Séquences N° 3 et N° 4, de Séquences N° 5 et N° 6, de Séquences N° 7 et N° 8, de Séquences N° 9 et N° 10, et de Séquences N° 11 et N° 12, et des moyens permettant d'identifier des amplicons spécifiques d'une espèce, comprenant une ou plusieurs représentation(s) de la courbe de dissociation thermique de chaque amplicon isolé obtenu à l'aide d'une paire d'amorces faisant partie dudit ensemble, et/ou une ou plusieurs représentation(s) d'une ou de plusieurs courbe(s) de dissociation thermique de mélanges connus d'amplicons obtenus à l'aide des six paires d'amorces constituant ledit ensemble.

3. Trousse conforme à la revendication 2, comportant en outre un composé fluorescent s'intercalant dans l'ADN.

4. Trousse conforme à la revendication 3, pour laquelle ledit composé fluorescent s'intercalant dans l'ADN est choisi parmi les suivants : SYBR^{®} GreenER, Fast SYBR^{®} Green, LCGreen Plus+, SYT09, EVA Green et SYBR^{®} Green I.

5. Trousse conforme à l'une des revendications 2 à 4, comportant en outre une ou plusieurs fraction(s) aliquote(s) de réactifs pour PCR.
